# EUROPEAN PATENT SPECIFICATION

(11) **EP 1 676 545 B1**
(45) Date of publication and mention of the grant of the patent: **17.02.2010**
(21) Application number: 05257968.7
(22) Date of filing: 22.12.2005
(51) Int. Cl.: A61F 2/01

(54) **Distal protection device with improved wall apposition**
Distaler Schutzfilter mit verbesserter Apposition zu den Wänden
Filtre de protection distal avec apposition au paroi améliorée

(30) Priority: 30.12.2004 US 26308
(43) Date of publication of application: 05.07.2006
(73) Proprietor: Cordis Corporation, Miami Lakes, FL 33014 (US)
(72) Inventor: Arguello, Edward, Miramar, FL 33027 (US); Park, Jin S., Parsippany, NJ 07054 (US); Wang, Huisun, Miramar, FL 33027 (US); Wijeratne, Lalith Hiran, Cooper City, FL 33328 (US); Widenhouse, Christopher William, Cincinnati, OH 45249 (US)
(74) Representative: Gover, Richard Paul

(56) References cited:
- EP-A- 1 386 624
- WO-A-02/43595
- US-A- 3 540 431
- US-A1- 2004 127 933
- US-A1- 2004 204 738
- US-B1- 6 652 554

## Description

The present invention relates to intravascular devices used to assist in medical treatment and procedures. More specifically, the present invention relates to a blood filtering system for preventing embolic material from migrating through a blood vessel during an intravascular procedure.

Atherosclerosis is a complex disease, being primarily a result of buildup in the arteries that may start as early as childhood and progress as one ages. Progression may be rapid in some people. Blood vessels may become completely occluded or more often narrowed and/or stenotic in a number of ways. A stenosis may be formed by an atheroma which is typically a harder, calcified substance which forms on the inside walls of the blood vessel. The stenosis may also be formed by a buildup of thrombus material, which may restrict blood flow through the vessel. In general, atherosclerosis is the result of any combination of fat substances, cholesterol, waste products, calcium as well as other substances being deposited on the inside lining of the artery. This buildup is often called plaque. Atherosclerosis can often lead to coronary heart disease, a major health concern today for both males and females in the United States as well as abroad.

Atherosclerosis may also lead to strokes, and other disorders because of the occurrence of blood clots, which may form in the narrowed arteries. Although plaques can grow large enough to significantly reduce the blood flow through an artery, most of the damage may occur when these plaques become fragile and rupture. These are often referred to as vulnerable plaques. When vulnerable plaques rupture they typically cause blood clots to form that may then subsequently block blood flow or break off and travel through the blood vessel to another part of the body. If either situation happens, the result may be a blocked blood vessel that supports and nourishes the heart, which may cause a heart attack. If a blood vessel that delivers blood to the brain is blocked, it may cause a stroke. If the blood supply to the legs is compromised, it may result in limb ischemia and in difficulty with walking and/or leg pain referred to as claudication, and may eventually cause gangrene.

The narrowing of an artery, also known as a stenotic lesion in the vasculature, has motivated medical professionals to develop a number of intravascular procedures which have evolved over time to treat this condition, percutaneous balloon angioplasty being the most common. Percutaneous balloon angioplasty is a procedure wherein a balloon catheter is inserted within the vasculature, and the balloon is expanded at the location of the lesion essentially compressing the stenotic buildup against the inside of the vessel wall. More recently this procedure has been augmented by the deployment of a stent or stents, at the location of the lesion subsequent to, or concurrently with the angioplasty. The stent acts as an internal scaffold within the vessel, retaining an open lumen and preventing further re-narrowing of the vessel. Generally, stents are primarily of two types, balloon expanding and self-expanding. As the terms indicate, balloon-expanding stents are deployed/expanded *in-vivo* with the assistance of a balloon, while self-expanding stents may utilize shape-memory materials such as nitinol. The use of such alloys as nitinol (Nickel-Titanium alloy), having shape memory characteristics, suitable biocompatibility, and designed to be inserted into one's vasculature, is known in the art. These so-called shape memory alloys have the ability to return to their original shape when exposed to the correct temperature conditions. The shape memory characteristics of these alloys, allow the devices to be deformed to facilitate their insertion into a body lumen or cavity. Upon being exposed to higher temperatures within the body results in the device returning to its original programmed shape. Thus one can employ the shape memory property to expand the device to its original shape after being delivered through the vasculature in a reduced profile or compressed state. Nitinol can also have super-elastic characteristics, which allow the device fabricated from such a material as nitinol, to be deformed and restrained in the deformed condition in order to facilitate the insertion into a patient's vasculature. This deformation causes a phase transformation of the material. Once the device with super-elastic characteristics is delivered, the restraint on the super-elastic material can be removed thus reducing the stress and allowing the previously compressed super-elastic member to return to its original pre-programmed and un-deformed shape, which results in a transformation back to the original phase.

While widespread intravascular procedures, particularly angioplasty procedures have been extremely successful; the procedure itself may result in development of an embolus. For example, during stent deployment and positioning, abrasion of the vessel wall may dislodge material resulting in an embolus. An embolus circulating within the blood vessels may lead to occlusion of a vessel and/or formation of clots within the vasculature and/or body organs. Although the occurrence of this can be minimized with careful and proper technique, when such an event does happen it may have serious consequences to the patient. By adequately capturing and/or filtering the material traveling in the blood responsible for causing such an event one can avoid the serious consequences that may result without such safeguards.

Multiple approaches to address capturing and/or filtering of the embolic debris/material from blood have been attempted. These include baskets, nets, suction, and even chemical modification of the debris (see US-5053008, which utilizes and requires an additional conduit to transport lysing agents to the trapped embolus). Use of vascular filters in the Vena Cava for capturing emboli has been disclosed (see US-4727873 and US-4688553). The designs of the Vena Cava filters continue to improve addressing such issues as fit and preventing migration (see US-6443972). Distal Protection devices using filtering baskets although similar to Vena Cava filters in function are typically temporarily positioned within the lumen, whereas Vena Cava filters are typically implanted within the vessel, most often the inferior Vena Cava, as the name implies. The use of a filtering basket positioned downstream from the procedure to capture the debris/material during an intravascular procedure is one such method. Because these basket type devices must be introduced into the vasculature and travel within the vasculature to be ultimately positioned to a location somewhat distal or downstream to the region of interest, most, if not all, incorporate and utilize concepts and features that make the essential delivery through the vessel less traumatic (see US-6391044). This can be accomplished by using a reduced size or compressed version of the apparatus. This allows one to deploy the apparatus to its normal working size when the apparatus is at the location of interest within the vessel.

The majority of these basket-type devices employ "expandable filters," meaning they may be fabricated from shape-memory materials which have the property that when exposed to the relatively elevated temperature within the body, they return to their initial programmed size. Alternately, on can rely on the superelastic property by removing the restraint on the geometry. These devices are generally placed distal, or downstream of the stenosis in order to capture any fragments, debris, and/or embolic material, that may be dislodged or occur as a result of the presence and use of the device during an intravascular procedure. The downstream placement of the device takes advantage of the blood flow within the vasculature, which will transport the undesirable material with it. The filtering membrane of the device is typically designed so as to allow blood flow through the membrane while limiting passage of the larger sized fragments and debris such as micro and macro emboli. These fragments, debris, and/or embolic material could potentially be carried beyond the device location with the blood flow downstream if not for such a filtering device as described herein. While this method performs fairly well capturing a substantial portion of the items intended to be captured; many of these designs are optimized for circular vessels. While outer vessel shapes are circular or slightly elliptical, the internal geometry of a vessel may often be non-circular as in an oval or elliptical shape or may even take the form of other non-circular geometries. Specifically, when calcification is present within the vessel, the internal vessel geometry is often irregular. Furthermore, vessel cross-sectional shape may vary with the type and location of vessel and vary across patients as well. Moreover, due to the circulation of blood through the vessel and resulting forces, a dynamic situation exists, which may produce additional geometry changes to the normal vessel shape. Thus expandable filters, which are generally designed for circular vessels, may result in a lack of apposition against the vessel wall over at least some portion of the internal circumference of the vessel wall when one takes into account the additional factors described above. Such a gap or leak path may occur when the resulting geometry of the expanded device is circular while the inner luminal cross-sectional shape of the vessel is more often non-circular. Such resulting gaps, between the device and inner luminal surface, may allow the emboli that the device is designed to capture, adequate room to pass through such a gap between the inner wall of the vessel and the outer confines of the device. When this occurs, the primary purpose of the device, which in this case is to capture fragments, debris and/or embolic material, is defeated, because the unfiltered flow path will allow for passage of the emboli. Even when the vessel itself is circular, conformance of a circular filter to the internal lumen of the vessel may not be optimal if "in-folding" is present. "In-folding" is the situation when the unsupported membrane of the system folds in at positions located between the strut locations where the membrane is supported by the struts. This situation can produce gaps between the inner vessel wall and the membrane even in the idealized circular vessel at locations between adjacent struts. In-folding may also occur when an oversized device (one that is sized larger then the vessel it will be placed in) is utilized in order to ensure adequate vessel coverage. In this situation, when the struts of the oversized device make contact with the vessel wall, the device ceases to expand. As a result, this limited expansion is short of its fully expanded programmed size and as such the membrane is not fully taught. Thus the remaining slack present in the membrane may lead to in-folding and thus allow for an unfiltered flow path. Moreover, even in the absence of in-folding, utilization of a circular-type device in a truly circular vessel may not adequately conform to the internal lumen upon vessel loading and/or deformation because the resulting device deformation may not adequately match the deformation of the vessel. This dissimilar deformation may thereby result in gaps or leak paths between the inner vessel wall and the device upon loading and/or deformation of the vessel.

US 2003/0204738 discloses methods, devices and systems for extracting vessel occluding materials. The devices are provided with a plurality of expandable members, a slidable mechanism, and a circumferential member to which the expandable member is attached. US 2004/0127933 discloses an embolic protection device. The device includes a an elongate shaft and a filter.

Accordingly, there is a need for a distal protection device with improved filtering and vessel apposition that can allow for capturing of embolic debris regardless of vessel size or shape or various loading regimes encountered by the vessel.

The distal protection device with improved apposition in accordance with the present invention overcomes the disadvantages and shortcomings of currently available devices and satisfies the unmet needs of maximizing capture of embolic debris by improved vessel apposition in vessels of varying size and shape in various loading and no-load regimes.

The present invention relates to an apparatus for intravascular filtering, capable of capturing emboli in blood flowing within the vasculature and a method of using the device. The filtering device comprises an expandable basket and is configured to deploy radially outward which may be relative to a centrally located guide wire. Expansion of the filtering device with improved vessel conformance is accomplished in a fashion resulting in improved filtering of blood in both circular and non-circular vessels and for vessels both large and small as well as when the vessel itself encounters various internal and/or external loading regimes.. Furthermore the incorporation or application of biological and/or pharmaceutical agents can provide additional benefits when used in combination with the present invention.

In accordance with one exemplary embodiment of the present invention, the filtering basket and supporting struts work together to achieve the stated objective of improved conformance and apposition to the internal vessel wall. In this exemplary embodiment of the present invention where the filtering basket and struts work together, expansion of the filtering basket is decoupled from the struts, and as such, expansion of the filter basket can be independent of the expansion of the supporting struts that are operatively connected to the filtering basket. Operatively connected in this instance in accordance with an exemplary embodiments of the present invention equates to the struts connected to the membrane such that radial strut movement controls the radial expansion of the membrane but allows for independent longitudinal or axial movement of strut relative to the membrane. For example, in accordance with the present invention one can operatively attach the strut to the membrane by allowing the strut to slide within loops fixed to the membrane thereby allowing the radial expansion of the membrane to occur independent of the axial translation of the individual struts. The independent expansion of each of the supporting struts can independently act on the filtering basket enabling the basket to expand as well. This expansion of the filtering basket may be non-uniform. As a result, improved vessel wall apposition is achieved by the independent radial expansion of each of the struts acting upon the filtering portion of the device. This improved conformance to the inside surface of an vessel wall results in improved filtering capacity by capturing emboli and/or other material which may otherwise be allowed to circumvent a device which does not conform to the vessel wall as closely. This provides a significant patient benefit.

In yet another exemplary embodiment of the present invention, the struts themselves are constructed to be independent filtering elements that when acting together with other filtering elements create a filter basket with independent expansion relative to the adjacent strut/filter element. Operatively attached to each strut is one or more filtering flanges. The combined strut and filtering flange element interacts with adjacent strut and filtering flange elements creating a filtering cone with overlapping flanges to accommodate non-circular geometries and which are capable of expanding to the appropriate internal lumen or vessel diameter the device is positioned within.

In a fourth exemplary embodiment of the present invention, improved conformance in both circular and non-circular vessels is achieved and in-folding of the filtering aspect is addressed. In this exemplary embodiment of the present invention the path of a supporting strut is both axial and circumferential. The struts run axially in the distal portion of the filter and then each strut in succession is directed circumferentially at the midpoint of the filter for a limited portion of the circumference of the membrane and then each strut returns to an axial direction in the remaining proximal portion of the filter. This exemplary embodiment of the present invention allows for improved vessel conformance by providing support to the filter membrane along a substantial portion of the circumference of the device, located substantially within the middle third between proximal and distal ends of the device, which is in apposition with the lumen of the vessel wall. It is the circumferential portion of the strut acting upon the filtering membrane, which results in improved vessel conformance and which eliminates the occurrence of in-folding by providing support to the filtering membrane in apposition with the vessel wall over a significant portion of the circumference.

In a fifth exemplary embodiment of the present invention, one can improve conformance with the internal lumen of the vessel while further reducing the folded and/or compressed profile of the system by utilizing a series of independent struts optimized for improved apposition in both circular and non-circular vessels. This can be achieved by utilizing independent strut loops that occupy only the proximal portion of the filter thereby further reducing the compressed profile of the distal portion of the filter due to the absence of struts from the distal portion of the filter basket. Each independent strut loop can expand in a similar or dissimilar fashion relative to the additional strut loops, which allows for the improved conformance of the membrane to the vessel wall. In accordance with the present invention, examples of strut loops can include "U" and "V" type loops. The looping of the strut is achieved by and best described as a substantially axial portion of the strut, which is then directed circumferentially for a portion of the circumference, the path of the strut then following a second substantially axial portion returning to the approximate starting position of the strut and completing the loop. In "U" type loops, the circumferential portion of the loop in combination with the two axial portions can result in a shape similar to the letter "U", alternately in "V" type configurations, the distal axial portions of the strut may form a shape similar to the letter "V". The "V" type configuration, which facilitates easier and more efficient compression and/or folding of the device, due to the distal axial portions which can be aligned when compressed, results in reduced profile dimensions which is extremely important given the delivery considerations through the vasculature to the region of interest. The "V" type configuration may also be cut from smaller profile tubing. It is important to note that any suitable configuration can be utilized. The circumferential portion of the strut, which is operatively attached to the membrane, allows the extent of in-folding to be minimized while achieving improved vessel conformance, because the circumferential portion of the strut ensures conformance with the vessel wall by providing support to the membrane which is adjacent to the vessel wall. Since the struts in combination with the compressed or folded filtering membrane is the primary contributor to profile in the distal region of the device, limiting the strut loop to the proximal portion of the device minimizes the overall profile of the device due to the absence of struts in the distal portion.

The incorporation or application of biologically active or pharmaceutically active compounds with the present invention is a further object of this invention and is an improvement to methods and/or devices which require the use of a conduit to deliver the agent to the desired location. Compounds such as those identified below may be applied as coatings on these devices and may be used to deliver therapeutic and pharmaceutical agents which may include: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11_{b}/111ₐ inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

The use of compounds in conjunction with the present invention can provide distinct clinical advantages over existing therapies and/or devices. More specifically, compounds that are capable of causing lysis or degradation of the embolic debris can be incorporated into the filtering portion of the present invention. A factor to consider in the selection of such a compound is the origin of the debris be it thrombus, plaque, atheroma, or any other form representing an embolus. As the mesh and or pore size of the filtering aspect of the present invention decreases, more embolic material may become trapped in the filtering mechanism of the present invention, thereby increasing the load on the filtering portion. While small emboli (typically smaller than 100 microns) are not a major concern because of the body's natural ability to enzymatically degrade, digest or lyse the emboli, the embolic load on the filter itself can be overloaded and result in formation of a thrombus if the blood flow is significantly slowed to the point which allows for a thrombus formation. In this situation the incorporation or application of compounds, which can degrade trapped emboli, can be beneficial. Some exemplary suitable compounds may include: Tissue Plasminogen(TPA); Streptokinase(SK); Reteplase; Tenecteplase; Urokinase; Lanoteplase; Staphylokinase; and/or Nadroparin(anti-factor Xa). In addition, the filtering portion of the present invention may incorporate an antithrombotic and/or antithrombogenic agent to prevent the formation of a thrombus. Some exemplary compounds may include: Heparin; Fragmin (dalteparin, low MW Heparin); a monoclonal antibody such as ReoPro™ (abciximab, antiplatelet antibodies) Acenocoumarol; Anisindione; Dicumarol; Warfarin; Enoxaparin (Lovenox); Anagrelide (Agrylin); Indomethacin (Indocin); Dipyridamole; Clopidogrel; Aggrenox; and/or Coumadin. Furthermore, an affinity-binding compound may also be incorporated with the filtering aspect of the present invention by itself or in combination with other compounds. Affinity-binding compounds can promote the binding and/or adhesion of embolic material thus facilitating entrapment of embolic material and subsequent removal from the blood stream. Whether incorporated into the strut or membrane by methods such as chemical surface treatments, bombardment, placement into reservoirs, or in the case of polymeric struts and membranes, blended with the material itself, or by application of a coating to the struts and/or membranes with a compound, any identified compound or combination of identified compounds may be used. Furthermore any number of compounds may suggest themselves to one who is skilled in the art and may be utilized in connection with the present invention alone or in combination with other compounds.

The foregoing exemplary embodiments of the present invention each provide a reliable, easy to manufacture, and simple to use device that significantly improves wall apposition of the outer confines of the device to the internal surface of the vessel wall regardless of vessel size or shape or loading regime encountered. Furthermore, a relatively reduced profile of the delivered device is achievable in accordance with the present invention. Moreover, any combination of the items identified that are capable of expansion and provide for the ability to independently conform to both circular and non-circular geometries upon expansion may be utilized. As noted above, the incorporation of biological and/or pharmaceutically active agents with the present invention can be utilized for the additional purposes of preventing thrombus formation, promotion of binding, and degradation of thrombus, all of which provide a patient benefit.

Embodiments of the invention will now be described by way of example with reference to the accompanying drawings, in which:
Figures 1a & 1b are planar views showing the disparate cross-sectional coverage area of an existing prior art filtering device in both a circular vessel and a non-circular vessel and the resulting gap in coverage due to non-conformance of the device when positioned in non-circular vessels;
Figures 2a & 2b are planar views showing the disparate cross-sectional coverage area by a filter with improved vessel conformance in accordance with the present invention in both a circular and non-circular vessel. The schematic in figure 2b shows the minimization of gaps particularly in a non-circular vessel that results in contrast to the result achieved with prior art devices shown schematically in figure 1b;
Figure 3 is a partial side view of the filtering device in accordance with the present invention positioned on a guide wire;
Figure 4a is a partial side view of a conformal membrane-filtering basket without supporting struts in accordance with the present invention;
Figure 4b is a partial side view of the conformal membrane basket with stress relief to further improve vessel wall conformance in accordance with the present invention;
Figure 5 is a partial side view of the network of struts configured to create a conformal lattice, which serves as the filtering aspect in accordance with the present invention;
Figure 6a shows a partial side view of a filter basket with independent filtering elements in accordance with the present invention;
Figure 6b is a detailed view of a single independent filtering element of the filter basket as shown in figure 6a showing the strut and attached filter flange in accordance with the present invention;
Figure 7a is a three-dimensional perspective view showing an additional exemplary embodiment of a filtering device with improved vessel conformance in accordance with the present invention;
Figure 7b is a partial side view showing schematically the circumferential aspect of the struts as shown in figure 7a in accordance with the present invention;
Figure 7c is a partial side view showing the strut configuration, with the filter membrane removed for clarity, in accordance with the present invention;
Figure 7d is a partial side view of the embodiment shown in figure 7c with the filtering membrane attached in accordance with the present invention;
Figure 7e is a frontal view or head on view, as blood flow through the lumen would encounter with the membrane removed for clarity, in accordance with the present invention;
Figure 7f is a frontal view of the embodiment shown in figure 7e with the filtering membrane attached in accordance with the present invention;
Figures 8a & 8b are planar sectional views of both a device without support to the circumferential aspect resulting in the presence of in-folding, and of a device with the circumferential aspect of the filtering membrane supported by the strut resulting in improved vessel conformance in accordance with the present invention and where in-folding is minimized and/or completely eliminated because of the substantially circumferential 360 degree sealing of the filter membrane against the vessel wall;
Figure 9 is a partial side view showing an additional exemplary embodiment of a filtering device with improved vessel conformance and reduced profile in accordance with the present invention;
Figure 10a is a three-dimensional perspective view incorporating four strut loops of the "U" type configuration, operatively attached to a filtering membrane in accordance with the present invention;
Figure 10b is a three-dimensional perspective view incorporating three strut loops of the "U" type configuration operatively attached to a filtering membrane in accordance with the present invention;
Figure 11a is a partial side view of the four-loop embodiment of the "U" type configuration shown in figure 10a in accordance with the present invention;
Figure 11b is a partial side view of the four-loop embodiment of the "U" type configuration shown in figure 10a with additional filtering membrane support in accordance with the present invention;
Figure 11c is a partial side view of the four-loop embodiment of the "U" type configuration shown in figure 10a showing a scalloped membrane allowing for additional reductions in profile in accordance with the present invention;
Figure 11d is a partial side view of the four-loop embodiment of the "V" type configuration shown in figure 10a showing a scalloped membrane allowing for additional reductions in profile in accordance with the present invention; and
Figure 11e is a partial side view of the four-loop embodiment of the "U" type configuration shown in figure 10a showing both additional filtering membrane support and a scalloped membrane in accordance with the present invention.

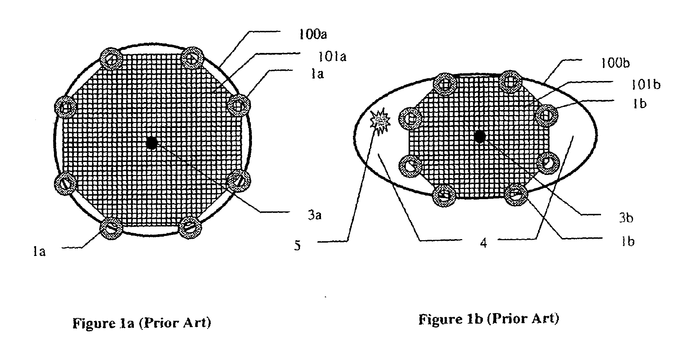

Figures 1a & 1b show a cross-sectional view for both a circular (100a) and a non-circular (100b) blood vessel. Superimposed within these vessels in figures 1a & 1b are schematics showing the approximated or relative cross-sectional coverage area (101a) and (101b) for typical existing prior art devices in both circular (100a) and non-circular (100b) idealized vessels. In this case the vessel wall (100a) & (100b) constrains the extent of the expansion of devices located within the circumference of the vessel. With existing devices that uniformly expand, this expansion is such that strut point locations represented by (1a) & (1b) in the circular and non-circular vessels respectively, are uniformly equidistant from the central axis (3a & 3b). As such, when strut point locations (1b) come into contact with the vessel wall (100b), at the moment one or more struts make contact, all subsequent expansion is halted. As shown, given that all strut point locations (1b) expand in unison and uniformly, the right two and left two strut point locations (1b) never make contact with the internal vessel wall surface (100b) because the top two and bottom two strut point locations (1b) make prior contact with the vessel wall (100b) and thus the entire device is prevented from further expansion. This results in a gap of coverage area (4) that may allow embolic material (5) to flow past the filtering device. The gap in coverage area is not only present in non-circular vessels, but depending on the extent of non-circularity of the vessel may result in further increasing the gap present and may also occur or increase due to various vessel loading situations.

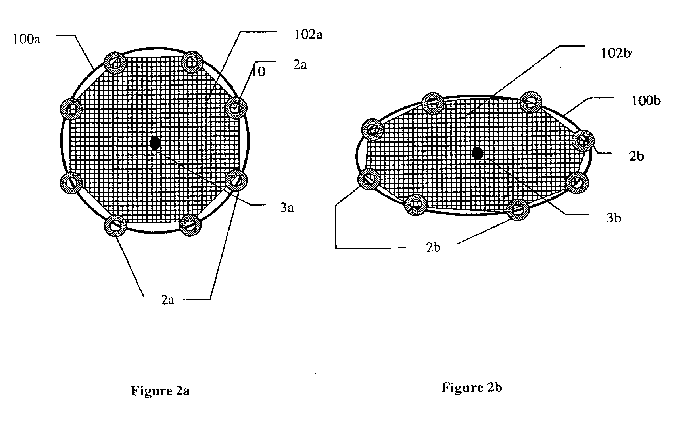

This is in contrast with the result achieved with an improved vessel conformance device in accordance with the present invention. A similar set of schematics represented in Figures 2a & 2b show the cross-sectional coverage area and obtainable results in accordance with the present invention in both circular (100a) and non-circular (100b) vessels. Figures 2a & 2b show cross-sectional views for both a circular (100a) and a non-circular (100b) blood vessel. Superimposed within this vessel is a schematic showing the cross-sectional coverage area (102a) and (102b) for an improved vessel conformance devices in accordance with the present invention in both circular (100a) and non-circular (100b) vessels. In this case the vessel wall (100a) & (100b) constrains the extent of the expansion of devices located within the circumference of the vessel as before. However because the strut point locations (2a & 2b) are decoupled from the filtering portion these strut points can continue to expand independently of each other and the filtering basket, even when one strut point location makes contact with the vessel wall. Thus the outward expansion of the other strut points (2b) are not inhibited and thus can continue to expand until each independently makes contact with the vessel wall regardless of the nature of the cross-sectional shape of the vessel. This results in minimization and/or avoidance of any gap in coverage area.

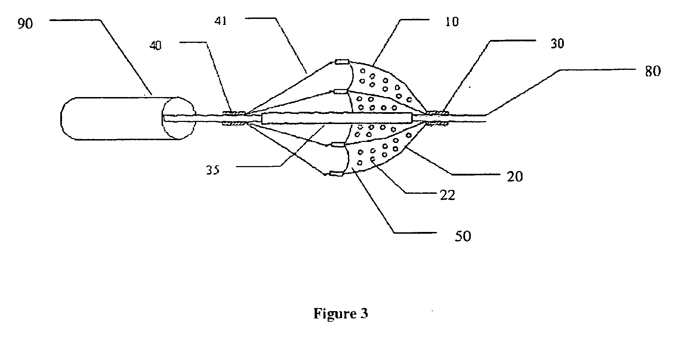

Figure 3 shows a side view of an exemplary embodiment of the present invention, positioned on a guide wire (80). The filter basket (10) includes one or more struts (20), which serve to provide support and impart shape to the filter membrane (50). The filter membrane (50) in this exemplary embodiment is shown with uniform holes (22), but can also have a distribution of varying diameter holes to improve filtering performance or optimize filtering performance to achieve the desired result. Any number of designs may suggest themselves to one who is a skilled artisan in the field and may be utilized in connection with the present invention. The distal ends of the struts (20) are pivotally attached to supporting collar (30), which may be permanently, removably, or operatively attached to guide wire (80). When operatively attached, supporting collar (30) can slide and/or rotate with respect to guide wire (80). The struts (20) are operatively attached to the filter membrane (50) which taken together form the filter basket (10). The distal ends of additional tension members (41), attach to proximal ends of struts (20), while the proximal ends of these tension members (41) are fixed to the closure ring (40). When proximal movement (ie: away from supporting collar (30)) is imparted to closure ring (40) this increases the tension in tension members (41) such that strut members (20) are pulled radially inward toward guide wire (80) allowing for filter basket (10) comprised of filter membrane (50) and struts (20) to be collapsed with any retained debris to be captured in basket. The entire assembly can then be pulled back into retaining sheath (90). An inner sleeve (35) whose axial position relative to guide wire (80) can impart motion to either closure ring (40) or supporting collar (30) in order to draw the assembly into the retaining sheath (90) or to deliver the assembly from the retaining sheath (90) may also be present. The pore size of filter membrane (50) can be optimized by altering the diameters of the individual pores (22) as well as their location of different sized pores relative to each other in order to maximize optimal blood flow through the membrane (50) while still filtering debris for whose size is of consequence. Preferred pore size diameters of individual pores (22) of the present invention range from approximately 50 to 150 µm (50 to 150 microns), however any number of suitable combinations of large and small size pores is also possible as is the distribution of said pores with respect to the filter membrane (50).

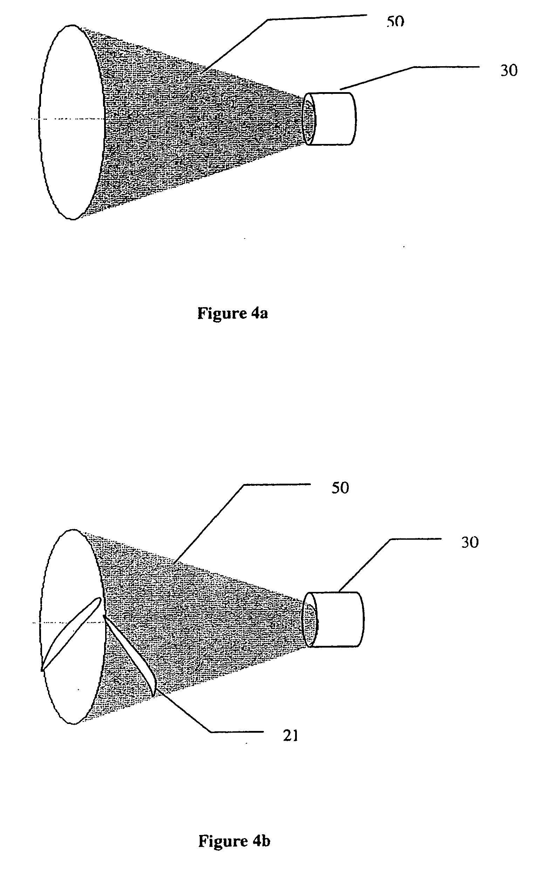

Figure 4a shows a partial side view of an additional exemplary embodiment of the present invention. Here, the filter basket (50) is a flexible expanding conical membrane. This membrane can be constructed from various materials such polyurethane and/or nitinol. Nitinol thin film is particularly advantageous because of its appropriate mechanical properties coupled with its reduced profile. The skilled artisan will recognize that any number of suitable biocompatible materials may be employed for this purpose. The distal end of the filter membrane is operatively attached to the proximal face of the supporting collar (30). The membrane can be constructed with geometric relief (21), as shown in Figure 4b to provide adequate flexibility, minimal thickness and/or combinations of any of the afore mentioned items in order to maximize conformability to non-circular lumen cross-sections (100b).

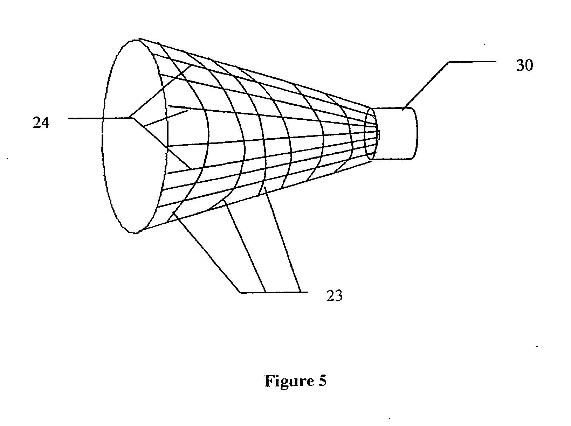

Figure 5 shows a partial side view of the filter basket configured from longitudinal (24) and circumferential (23) struts alone without the presence of a membrane to allow for adequate expansion in order to conform to non-circular vessels (100b). In this embodiment the struts (23 & 24) are combined to form a lattice, which can then serve as both the structural support as well as the filtering aspect without the use of a filtering membrane. The circumferential aspect may be a single helically wrapped strut that runs substantially perpendicular to the multiple longitudinal struts (24) or a combination of multiple struts substantially circular in geometry that are free to expand radially and substantially parallel to each other and substantially perpendicular to the longitudinal struts (24).

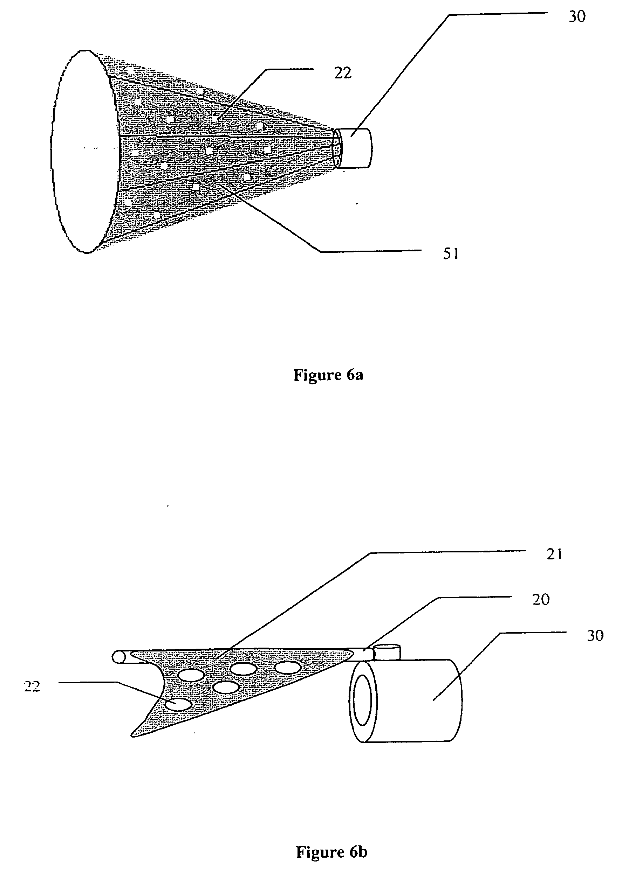

Figures 6a & 6b show an alternate exemplary embodiment of the present invention in which multiple independent filter flanges (21) are connected to independent struts (20), which are operatively attached to the proximal face of the supporting collar (30) or in an alternate fashion to the external circumference of the supporting collar (30) or in an additional alternate fashion to the inner aspect of the supporting collar (30) in order to combine to form a nested filter basket (51) and are utilized to improve conformance with the internal vessel the device is located within. The detail view of figure 6b shows an isometric view of a single member of an additional exemplary embodiment of the invention utilizing filter flanges (21) as shown in figure 6a. In this exemplary embodiment the filtering flange (21) is attached to the independent strut (20) whose distal end is attached to supporting collar (30). Through holes (22) are shown on filter flange (21) to allow for blood to pass through while maintaining adequate filtering capacity to capture the debris. The through holes (22) can vary in size but are preferably between 50 and 150 µm (50 and 150 microns). In addition the distribution and number of through holes (22) can be varied as well. Several of these strut combination-filtering flanges would operate in concert with each to create a filtering basket (51) as shown in figure 6a. Conformity to non-circular vessels (100b) is once again achieved by independent expansion of each strut/filtering flange combination.

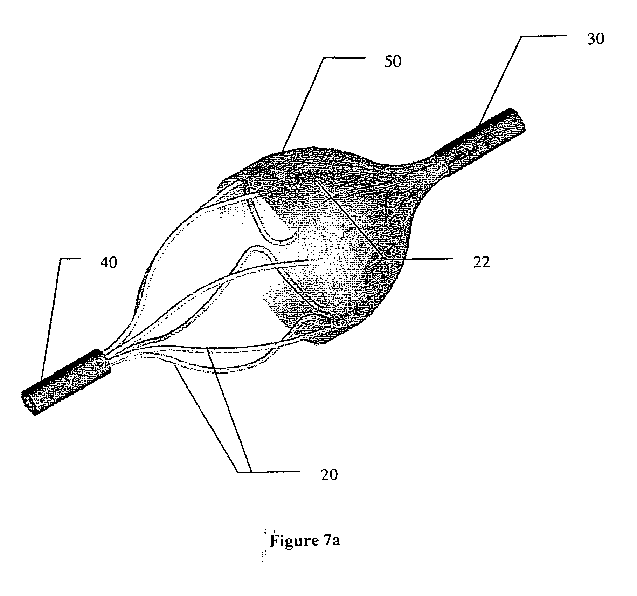

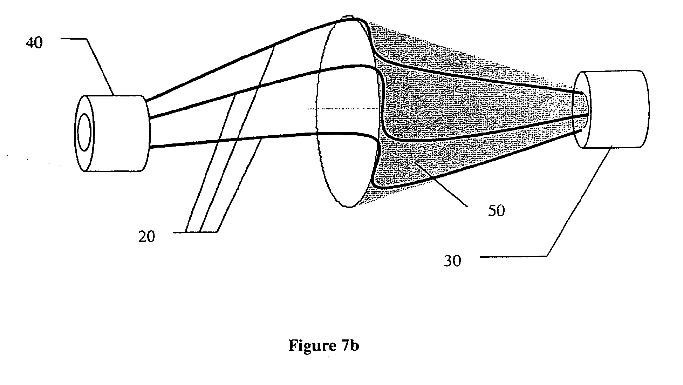

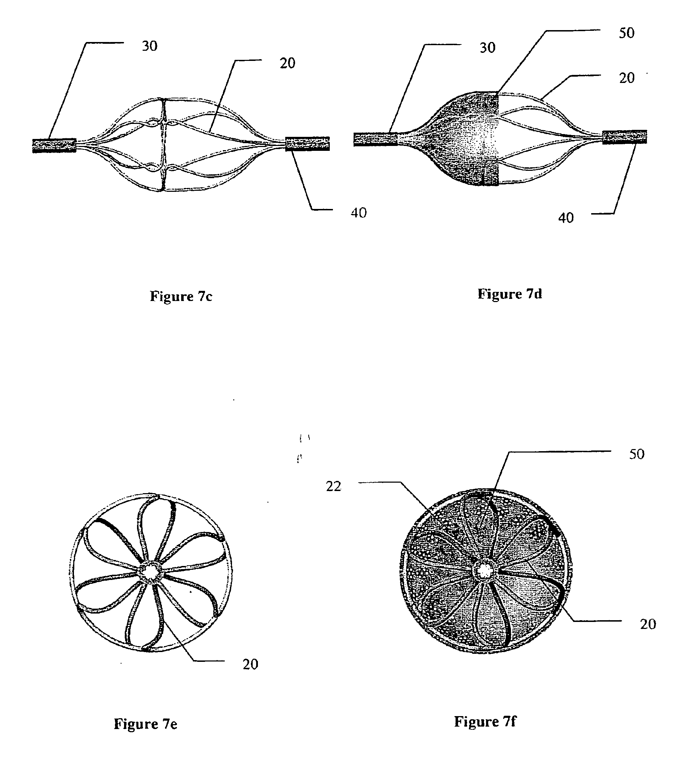

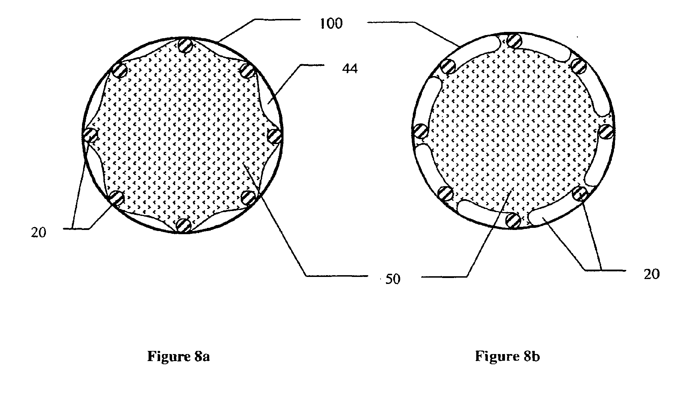

Figures 7a & 7b show an isometric view of an additional exemplary embodiment of the present invention, which is characterized by each of the individual strut paths (20) that run or are aligned both axially and circumferentially. In this exemplary embodiment of the present invention, the distal end of the individual strut (20) is attached to the supporting collar (30) and is substantially aligned with the guide wire (80) (not shown in figure 7a & 7b) in a substantially axial direction. Approximately mid-way or in the middle third of the device but distal to the proximal opening of the filter basket (50) the strut (20) is directed circumferentially for a portion of the circumference of the filter basket only to be aligned with the guide wire (80) (not shown in this figure) once more as the proximal end of the strut (20) is again directed axially which then terminates at the supporting collar (40). This configuration of the strut (20), in particular the circumferential portion of the strut (20), allows for improved apposition of the filter basket (50) to the internal circumference of the lumen vessel wall (100) as shown in Figures 8a & 8b. Figure 8(a) shows a typical cross-section of the vessel wall with a filter membrane (50) supported solely by the axial portion of eight struts (20). The presence of in-folding between adjacent struts is apparent resulting in a gap (44) between the filter basket (50) and vessel wall (100) that can allow for passage of emboli. Figure 8(b) shows a similar cross-section of the vessel wall (100) with a filter membrane (50) supported by the struts (20) that for a portion of the circumference are directed circumferentially. As figure 8(b) shows, the gap due to in-folding is eliminated due to the additional support provided by the circumferential portion of the strut (20) to the filter membrane (50) against the vessel wall (100). By increasing the length of the circumferential portion of the struts (20) one can effectively reduce the number of struts (20) required to provide support to the filter membrane (50) without any decrease in apposition of the filter basket/membrane to that of the interior of the vessel wall (100). This decrease in the number of struts (20) can result in additional reductions in profile of the overall device thus allowing for delivery to smaller vessels. Figures 7c & 7d show a side view of this exemplary embodiment of the present invention both with and without the filter membrane (50). Figures 7e & 7f show frontal views both with and without the filter membrane (50) in which one exemplary configuration of filtering holes (22) of the present invention can be seen as shown in figure 7f.

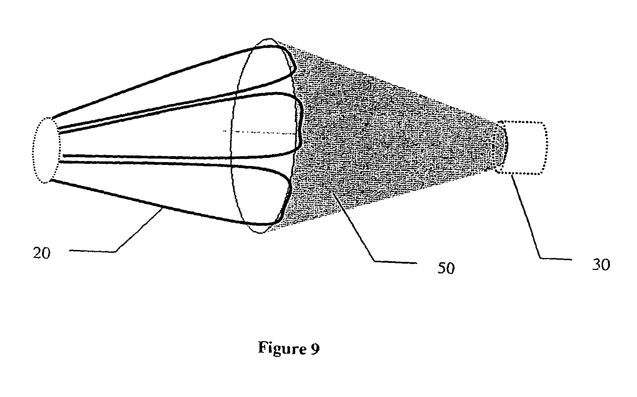

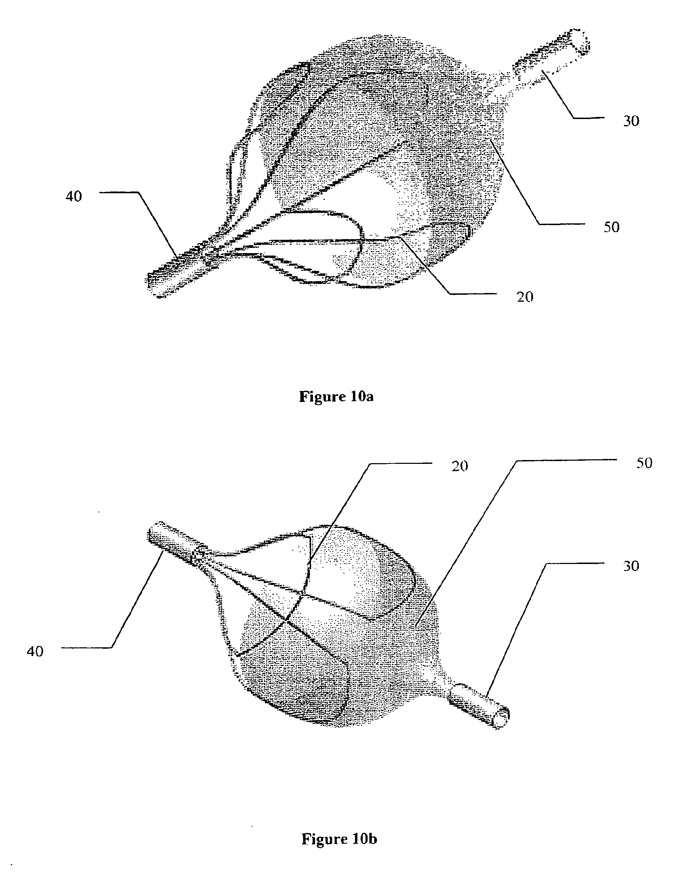

Figure 9 shows an alternate exemplary embodiment of the present invention for whose compressed profile is further reduced by the omission of struts (20) from the distal portion of the filter basket (50). As shown in figure 9 as well as in figures 10a & 10b, the struts (20) begin from a position proximal from the filter basket and are directed substantially axially until they reach the proximal opening of the filter basket (50) at which point they are directed circumferentially for a portion of the circumference of the filter basket (50) and then redirected back in the axial direction to the proximal starting position thus forming the substantial portion of a loop. This is repeated for each strut present, which allows for improved conformance of the filter basket's proximal opening to that of the vessel wall (100) in a similar fashion as was accomplished in the previous embodiment without adding additional profile to that of the filter basket (50) in the distal region due to the absence of struts (20) in the distal region. In this exemplary embodiment of the present invention, the distal supporting collar (30) is optional as the strut loops serve both to provide shape and support to the filter basket (50). As an example, the filter basket may be substantially spherical in shape or parabolic. Thus the filter basket can be formed into a net or parachute shape without the need for the distal supporting collar (30), or alternately a supporting collar (30) can be attached if additional control of the filter basket (50) is desired.

Figures 10a and 10b represent examples of two specific embodiments in accordance with the present invention with said supporting collar (30) present. Figure 10a is a four-loop configuration comprised of four independent strut loops (20). While figure 10b represents a three-loop configuration comprised of three strut loops (20). For each strut loop (20) the terminal starting and ending points of the loop (20) are fixed to the proximal support ring (40) while the distal circumferential portion of each respective loop is fixed to the proximal opening of the filter basket (50). In this exemplary embodiment, the distal support collar (30) is optional in the configurations shown. Furthermore, although not shown, filter membrane (50) can be optimized for filtering capacity by incorporating a combination of pores with consistent or varying sizes and distributions. In each exemplary embodiment, the device including both loops (20) and proximal support ring (40) may be cut from a single tube eliminating the need for separate structural components. As an example, laser cutting techniques for stent manufacturing can be employed to fabricate the embodiments described in accordance with the present invention. Cutting all or most of the structural components from a single tube by laser cutting or other appropriate methods provides significant cost savings as a result of the reduced number of manufacturing process steps. In certain instances, formed wire may also be used to fabricate the device in accordance with the present invention. Some device designs and shapes simply do not lend themselves to cost effective laser cutting and thus wire forming would be more cost effective. Supporting struts (20) can be fabricated from a number ofbiocompatible materials including metals, ceramics, and polymers. Preferable materials for the supporting struts (20) are shape memory metals and super-elastic alloys such as nitinol.

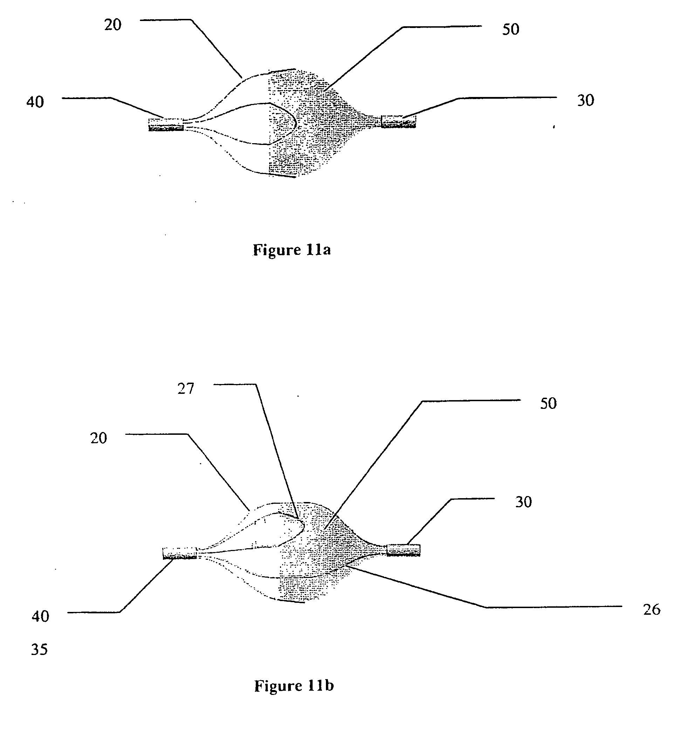

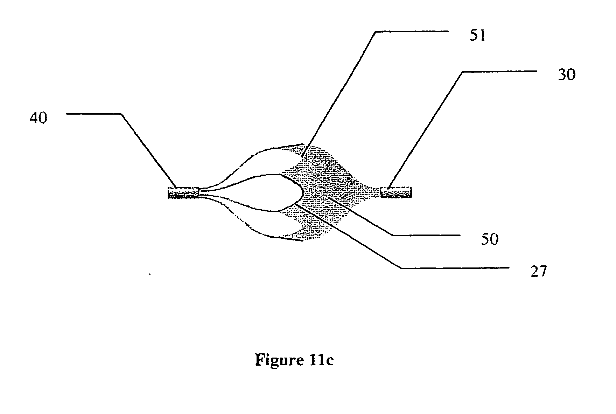

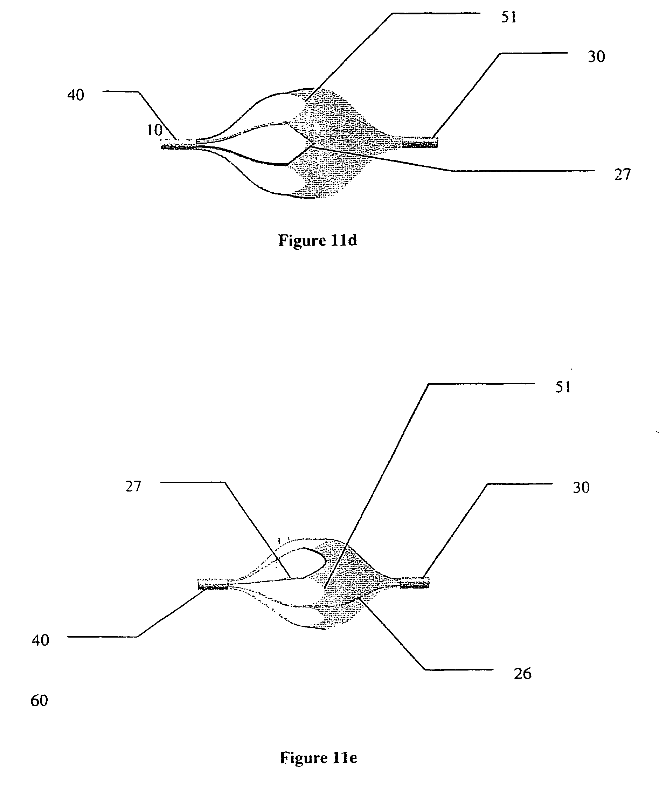

Additional embodiments are shown in figures 11a through 11e in which the three or four loop configuration can be augmented by providing additional filter support accomplished by strut member (26) as shown in figures 11b & 11e. These embodiments of the present invention are also capable of being fabricated from a single tube. Preferably these additional intermediate strut members (26) would be located between the struts having the loop configuration (27). Alternately, the strut loop configuration (27) can be a "U" type configuration as shown in figure 11c or a "V" type configuration as shown in figure 11d. Alternately, the membrane (50) can be scalloped (51) as shown in figures 11c, 11d & 11e, which can result in additional profile reductions without any decrease in filtering effectiveness.

## Claims

1. A vascular filtering device comprising:
a supporting collar (30) having a concentric though hole adapted to slidably engage with a guide wire (80);
primary supporting struts (20), each of said struts (20) having a proximal end and a distal end wherein the distal end of said struts is operatively attached to said supporting collar (30); and
a filtering flange (50) having at least one through hole (22) attached to said supporting struts (20) between the proximal and the distal ends of said supporting, struts (20); **characterised in that** said primary supporting struts (20) can move radially independently of each other.

2. The filtering device of claim 1 further comprising one or more additional supporting struts (20) each having a filtering flange (21) operatively attached to said additional supporting strut (20) between the proximal and the distal end of said additional supporting strut (20) and spaced apart from said primary supporting strut (20) wherein the distal end of said additional supporting strut (20) is operatively attached to said supporting collar (30) and the proximal end of said additional supporting strut (20) is free to expand away from the guide wire (80).

3. The filtering device of claim 1 further comprising:
a closure ring (40) having a concentric though hole adapted to slidably engage with the guide wire (80) positioned proximally to said supporting collar (30); and
a tension member (41) having a proximal end and a distal end wherein the proximal end of said tension member (41) is attached to said closure ring (40) and the distal end of said tension member (41) is attached to the proximal end of said primary supporting strut (20).

4. The filtering device of claim 2 further comprising:
a closure ring (40) having a concentric though hole adapted to slidably engage with the guide wire (80) positioned proximity to said supporting collar (30); and
at least one tension member (41) having a proximal end and a distal end wherein the proximal end of said tension member (41) is attached to said closure ring (40) and the distal end of said tension member (41) is attached to the proximal end of said primary supporting strut (20).

5. The filtering device of claim 1 wherein said filtering flange (21, 40) is a Nickel-Titanium Alloy thin film.

6. The filtering device of claim 2 wherein said filtering flanges (21, 40) are Nickel-Titanium Alloy thin film.

7. The filtering device of claim 1 wherein said filtering flange (21, 40) is a polymeric film.

8. The filtering device of claim 2 wherein said filtering flanges (21, 40) are a polymeric film.

9. The filtering device of claim 7 wherein said polymeric film is polyurethane.

10. The filtering device of claim 8 wherein said polymeric film is polyurethane.

11. The filtering device of claim 1 wherein said strut (20) is metal.

12. The filtering device of claim 2 wherein said struts (20) are metal.

13. The filtering device of claim 1 wherein said filtering flange (21, 40) incorporates an active compound.

14. The filtering device of claim 2 wherein said filtering flanges (21, 40) incorporate an active compound.

15. The filtering device of claim 1 wherein said strut (20) incorporates an active compound.

16. The filtering device of claim 2 wherein said struts (20) incorporate an active compound.

17. The filtering device of claim 1 or 2 wherein said strut (20) is polymeric.

18. The filtering device of claim 1 or 2 wherein said strut (20) is ceramic.

19. The filtering device of claim 11 or 12 wherein said metal is a shape-memory alloy.

20. The filtering device of claim 11 or 12 wherein said metal is a super-elastic alloy.

21. The filtering device of claim 19 wherein said shape-memory alloy is Nickel-Titanium Alloy.

22. The filtering device or claim 20 wherein said super-elastic alloy is Nickel-Titanium Alloy.

## Patentansprüche

1. Vaskuläre Filtervorrichtung, welche Folgendes umfasst:
einen Stützkragen (30), welcher ein konzentrisches Durchgangsloch aufweist, welches geeignet ist, um verschiebbar mit einem Führungsdraht (80) einzukoppeln,
primäre Stützstreben (20), wobei jede Strebe (20) ein proximales Ende und ein distales Ende aufweist, wobei das distale Ende der Streben wirksam an dem Stützkragen (30) befestigt ist; und
einen Filterflansch (50), welcher zumindest ein Durchgangsloch (22) aufweist, und
welcher an den Stützstreben (20) zwischen den proximalen und den distalen Enden der Stützstreben (20) befestigt ist, **dadurch gekennzeichnet, dass** sich die primären Stützstreben (20) radial unabhängig voneinander bewegen können.

2. Filtervorrichtung nach Anspruch 1, welche weiterhin eine oder mehrere zusätzliche Stützstreben (20) umfasst, wobei jede einen Filterflansch (21) aufweist, welcher wirksam an der zusätzlichen Stützstrebe (20) zwischen dem proximalen und dem distalen Ende der zusätzlichen Stützstrebe (20) befestigt ist und von der primären Stützstrebe (20) beabstandet ist, wobei das distale Ende der zusätzlichen Stützstrebe (20) wirksam an dem Stützkragen (30) befestigt ist, und das proximale Ende der zusätzlichen Stützstrebe (20) frei ist, um von dem Führungsdraht (80) weg zu expandieren.

3. Filtervorrichtung nach Anspruch 1, welche weiterhin Folgendes umfasst:
einen Verschlussring (40), welcher ein konzentrisches Durchgangsloch aufweist, welches geeignet ist, um verschiebbar mit dem Führungsdraht (80) einzukoppeln, welcher proximal zu dem Stützkragen (30) positioniert ist, und
ein Zugglied (41), welches ein proximales Ende und ein distales Ende aufweist, wobei das proximale Ende des Zuggliedes (41) an dem Verschlussring (40) befestigt ist, und
das distale Ende des Zuggliedes (41) an dem proximalen Ende der primären Stützstrebe (20) befestigt ist.

4. Filtervorrichtung nach Anspruch 2, welche weiterhin Folgendes umfasst:
einen Verschlussring (40), welcher ein konzentrisches Durchgangsloch aufweist, welches geeignet ist, um verschiebbar mit dem Führungsdraht (80) einzukoppeln, welcher proximal zu dem Stützkragen (30) positioniert ist, und
zumindest ein Zugglied (41), welches ein proximales Ende und ein distales Ende auf weist, wobei das proximale Ende des Zuggliedes (41) an dem Verschlussring (40) befestigt ist, und das distale Ende des Zuggliedes (41) an dem proximalen Ende der primären Stützstrebe (20) befestigt ist.

5. Filtervorrichtung nach Anspruch 1 wobei der Filterflansch (21, 40) ein dünner Nickel-Titan-Legierungsfilm ist.

6. Filtervorrichtung nach Anspruch 2, wobei die Filterflansche (21, 40) ein dünner Nichel-Titan-Legierungsfilm sind.

7. Filtervorrichtung nach Anspruch 1, wobei der Filterflansch (21, 40) ein Polymerfilm ist.

8. Filtervorrichtung nach Anspruch 2, wobei die Filterflansche (21 ,40) ein Polymerfilm sind.

9. Filtervorrichtung nach Anspruch 7, wobei der Polymerfilm Polyurethan ist.

10. Filtervorrichtung nach Anspruch 8, wobei der Polymerfilm Polyurethan ist.

11. Filtervorrichtung nach Anspruch 1, wobei die Strebe (20) Metall ist.

12. Filtervorrichtung nach Anspruch 2, wobei die Streben (20) Metall sind.

13. Filtervorrichtung nach Anspruch 1, wobei der Filterflansch (21, 40) eine aktive Verbindung enthält.

14. Filtervorrichtung nach Anspruch 2, wobei die Filterflansche (21, 40) eine aktive Verbindung enthalten.

15. Filtervorrichtung nach Anspruch 1, wobei die Strebe (20) eine aktive Verbindung enthält.

16. Filtervorrichtung nach Anspruch 2, wobei die Streben (20) eine aktive Verbindung enthalten.

17. Filtervorrichtung nach Anspruch 1 oder 2, wobei die Strebe (20) polymer ist.

18. Filtervorrichtung nach Anspruch 1 oder 2, wobei die Strebe (20) keramisch ist.

19. Filtervorrichtung nach Anspruch 11 oder 12, wobei das Metall eine Formgedächtnislegierung ist.

20. Filtervorrichtung nach Anspruch 11 oder 12, wobei das Metall eine superelastische Legierung ist.

21. Filtervorrichtung nach Anspruch 19, wobei die Formgedächtnislegierung eine Nickel-Titan-Legierung ist.

22. Filtervorrichtung nach Anspruch 20, wobei die superelastische Legierung eine Nickel-Titan-Legierung ist.

## Revendications

1. Dispositif de filtrage vasculaire comprenant :
➢ un collier de support (30) qui présente un trou traversant concentrique adapté pour venir en prise de façon coulissante avec un fil guide (80) ;
➢ des entretoises de support (20) primaires, chacune desdites entretoises (20) présentant une extrémité proximale et une extrémité distale dans lesquelles l'extrémité distale desdites entretoises est fixée de manière opérationnelle audit collier de support (30) ; et
➢ une bride de filtrage (50) qui présente au moins un trou traversant (22) fixé auxdites entretoises de support (20) entre les extrémités proximales et distales desdites entretoises de support (20) ;
**caractérisé en ce que** lesdites entretoises de support (20) peuvent se déplacer de manière radiale indépendamment les unes des autres.

2. Dispositif de filtrage selon la revendication 1, comprenant en outre une ou plusieurs entretoises de support (20) supplémentaires, chacune d'elles présentant une bride de filtrage (21) fixée de manière opérationnelle à ladite entretoise de support (20) supplémentaire entre l'extrémité proximale et l'extrémité distale de ladite entretoise de support (20) supplémentaire et espacée de ladite entretoise de support (20) primaire, dans lequel l'extrémité distale de ladite entretoise de support (20) supplémentaire est fixée de manière opérationnelle audit collier de support (30) et l'extrémité proximale de ladite entretoise de support (20) supplémentaire est libre de s'étendre en allant en s'éloignant du fil guide (80).

3. Dispositif de filtrage selon la revendication 1, comprenant en outre:
> un anneau de fermeture (40) qui présente un trou traversant concentrique adapté pour venir en prise de façon coulissante avec le fil guide (80) placé de manière proximale par rapport audit collier de support (30) ; et
➢ un élément de tension (41) qui présente une extrémité proximale et une extrémité distale dans lequel l'extrémité proximale dudit élément de tension (41) est fixée audit anneau de fermeture (40) et l'extrémité distale dudit élément de tension (41) est fixée à l'extrémité proximale de ladite entretoise de support (20) primaire.

4. Dispositif de filtrage selon la revendication 2, comprenant en outre :
➢ un anneau de fermeture (40) qui présente un trou traversant concentrique adapté pour venir en prise de façon coulissante avec le fil guide (80) placé de manière proximale par rapport audit collier de support (30) ; et
➢ au moins un élément de tension (41) qui présente une extrémité proximale et une extrémité distale dans lequel l'extrémité proximale dudit élément de tension (41) est fixée audit anneau de fermeture (40) et l'extrémité distale dudit élément de tension (41) est fixée à l'extrémité proximale de ladite entretoise de support (20) primaire.

5. Dispositif de filtrage selon la revendication 1, dans lequel ladite bride de filtrage (21, 40) est constituée d'une couche mince d'alliage de nickel - titane.

6. Dispositif de filtrage selon la revendication 2, dans lequel lesdites brides de filtrage (21, 40) sont constituées d'une couche mince d'alliage de nickel - titane.

7. Dispositif de filtrage selon la revendication 1, dans lequel ladite bride de filtrage (21, 40) est constitué d'une couche polymère.

8. Dispositif de filtrage selon la revendication 2, dans lequel lesdites brides de filtrage (21, 40) sont constituées d'une couche polymère.

9. Dispositif de filtrage selon la revendication 7, dans lequel ladite couche polymère est en polyuréthane.

10. Dispositif de filtrage selon la revendication 8, dans lequel ladite couche polymère est en polyuréthane.

11. Dispositif de filtrage selon la revendication 1, dans lequel ladite entretoise (20) est en métal.

12. Dispositif de filtrage selon la revendication 2, dans lequel lesdites entretoises (20) sont en métal.

13. Dispositif de filtrage selon la revendication 1, dans lequel ladite bride de filtrage (21, 40) incorpore un composé actif.

14. Dispositif de filtrage selon la revendication 2, dans lequel lesdites brides de filtrage (21, 40) incorporent un composé actif.

15. Dispositif de filtrage selon la revendication 1, dans lequel ladite entretoise (20) incorpore un composé actif.

16. Dispositif de filtrage selon la revendication 2, dans lequel lesdites entretoises (20) incorporent un composé actif.

17. Dispositif de filtrage selon la revendication 1 ou la revendication 2, dans lequel ladite entretoise (20) est constituée d'un polymère.

18. Dispositif de filtrage selon la revendication 1 ou la revendication 2, dans lequel ladite entretoise (20) est constituée d'une céramique.

19. Dispositif de filtrage selon la revendication 11 ou la revendication 12, dans lequel ledit métal est un alliage à mémoire de forme.

20. Dispositif de filtrage selon la revendication 11 ou la revendication 12, dans lequel ledit métal est un alliage super-élastique.

21. Dispositif de filtrage selon la revendication 19, dans lequel ledit alliage à mémoire de forme est un alliage de nickel - titane.

22. Dispositif de filtrage selon la revendication 20, dans lequel ledit alliage super-élastique est un alliage de nickel - titane.
